(19) Europäisches Patentamt
European Patent Office
Office européen des brevets

(11) **EP 2 682 774 B1**

(12) **EUROPEAN PATENT SPECIFICATION**

(45) Date of publication and mention
of the grant of the patent:
**17.06.2020 Bulletin 2020/25**

(51) Int Cl.:
*G01R 33/36* *(2006.01)*          *A61B 5/055* *(2006.01)*
*A61B 5/00* *(2006.01)*          *G01R 33/3415* *(2006.01)*

(21) Application number: **13174243.9**

(22) Date of filing: **28.06.2013**

(54) **Catheter with synthetic aperture MRI sensor**

Katheter mit einem MRT-Sensor mit synthetischer Apertur

Cathéter avec capteur d'IRM à ouverture synthétique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.07.2012 US 201213539524**

(43) Date of publication of application:
**08.01.2014 Bulletin 2014/02**

(73) Proprietor: **Biosense Webster (Israel) Ltd.**
**Yokneam, 2066717 (IL)**

(72) Inventors:
• **Govari, Assaf**
**34400 Haifa (IL)**
• **Altmann, Andres Claudio**
**34757 Haifa (IL)**
• **Schwartz, Yitzhack**
**34606 Haifa (IL)**
• **Ephrath, Yaron**
**35170 Karkur (IL)**
• **Beeckler, Christopher Thomas**
**Brea, CA 92821 (US)**

(74) Representative: **Small, Gary James**
**Carpmaels & Ransford LLP**
**One Southampton Row**
**London WC1B 5HA (GB)**

(56) References cited:
JP-A- 2003 325 475      US-A- 5 447 156
US-A1- 2003 231 789      US-B1- 6 317 091

• JIANG HUI ET AL: "Synthetic aperture technique used in intravascular imaging", PROCEEDINGS OF THE INTERNATIONAL CONFERENCE ON ACOUSTICS, SPEECH, AND SIGNAL PROCESSING (ICASSP). SPEECH PROCESSING 1. ADELAIDE, APR. 19 - 22, 1994; [PROCEEDINGS OF THE INTERNATIONAL CONFERENCE ON ACOUSTICS, SPEECH, AND SIGNAL PROCESSING. (ICASSP)],, vol. v, 19 April 1994 (1994-04-19), pages V/185-V/188, XP010133739, DOI: 10.1109/ICASSP.1994.389501 ISBN: 978-0-7803-1775-8
• "Phased Array Radar Antennas" In: Skolnik M. I.: "Radar Handbook", 1990, McGraw-Hill Publishing Company, USA, XP002714544, ISBN: 0-07-057913-X pages 7.6-7.23, * chapter "Scanning of Arrays" *

**Description**

**FIELD OF THE INVENTION**

The present invention relates generally to magnetic resonance imaging of a patient, and specifically to enhancing the imaging using a probe inserted into the patient.

**BACKGROUND OF THE INVENTION**

Magnetic resonance imaging (MRI) is an extremely powerful technique for visualizing tissue, particularly soft tissue, of a patient. The technique relies on exciting nuclei, typically hydrogen nuclei, from their equilibrium state, and measuring the resonant radio-frequency signals emitted by the nuclei as they relax back to equilibrium. While present-day MRI systems may provide good images, any system for enhancing the images would be advantageous.

JP-A-2003 325475 relates to a catheter which is equipped with a plurality of RF antennas placed inside the catheter for active tracking and depicting tissues close to the catheter and in a wide field of view. Each of the antennas is configured to detect signals from a different region.

**SUMMARY OF THE INVENTION**

An embodiment of the present invention provides a medical probe according to claim 1.

Typically, the coils are planar, and respective planes of the coils define a common plane. In one embodiment the tissue lies in the common plane so that the signals generated by the coils are a maximum.

In a disclosed embodiment the processor is configured to determine the phase delay responsive to a direction of the tissue with respect to the distal end.

In a further disclosed embodiment the processor is configured to determine the phase delay responsive to a location of the tissue with respect to the distal end.

In a yet further disclosed embodiment the probe includes a position sensor located in the distal end, and the processor is configured to determine a position of the distal end in response to a position signal from the position sensor and to determine the phase delay responsive to the position.

In an alternative embodiment at least one of the coils is configured to provide a position signal for the distal end, and the processor is configured to determine a position of the distal end in response to the position signal and to determine the phase delay responsive to the position.

In a further alternative embodiment the spatially separated coils are positioned on a straight line. Alternatively, the spatially separated coils are positioned on a curved line.

In a yet further alternative embodiment the coils are separated equidistantly.

In another alternative embodiment the probe includes a robotic drive compatible with a magnetic resonance imaging (MRI) environment and configured to robotically insert the flexible insertion tube into the body cavity.

There is also provided, according to a further embodiment of the present invention, a medical probe in accordance with claim 11, which contains two arrays of planar coils.

Typically, at least one first planar coil and at least one second planar coil have a common center. In one embodiment the respective planes of the first planar coils are common to the first plane.

The present invention will be more fully understood from the following detailed description of the embodiments thereof, taken together with the drawings in which:

**BRIEF DESCRIPTION OF THE DRAWINGS**

Fig. 1 is a schematic, pictorial illustration of a system for enhanced magnetic resonance imaging (MRI), according to an embodiment of the present invention;

Fig. 2 is a schematic figure illustrating a distal end of a probe in cross-section, according to an embodiment of the present invention;

Fig. 3 is a schematic figure illustrating the distal end in cross-section, according to an alternative embodiment of the present invention;

Fig. 4 is a schematic figure illustrating an alternative distal end of the probe, according to an embodiment of the present invention; and

Fig. 5 is a schematic figure illustrating a further alternative distal end of the probe, according to an embodiment of the present invention.

## DETAILED DESCRIPTION OF EMBODIMENTS

### OVERVIEW

In an embodiment of the present invention a medical probe, typically a catheter, comprises a flexible tube having a distal end for insertion into a body cavity of a patient. The probe is configured to be used during a procedure using magnetic resonance imaging (MRI) that is performed on the patient. An array of spatially separated coils is positioned within the distal end, the coils typically being oriented within the distal end so that planes of the coils are located in a single plane common to all the coil planes. Typically, although not necessarily, the coils are equidistantly spaced from one another.

During the procedure, nuclei, typically hydrogen nuclei, are excited from their equilibrium state and undergo magnetic resonance, emitting radio frequency (RF) signals as they relax back to equilibrium. The signals are detected by receiving coils (external to the patient) in an MRI scanner used during the procedure, and are used to image the patient. In addition, a processor operates the coils within the distal end as a phased array of antennas, applying differing phase delays to the coils so as to maximize signals from a particular direction, or from a particular location, measured with respect to and in proximity to the distal end. The processor may analyze the signals to image tissue in the particular direction or from the particular location.

Typically, the processor uses the signals from the distal end phased array of coils to enhance the image, of the region around the distal end, formed by the MRI scanner receiving coils. The enhancement may take the form of an increased resolution, a faster imaging time, and/or an improved physical or chemical tissue differentiation of the image. Such enhancement improves the ability of an operator performing the procedure to judge the progress of the procedure. For example, if the MRI is applied during an ablation procedure on the heart, the enhanced MRI image may provide a more accurate measure of the temperature of tissue being ablated, compared to unenhanced MRI images.

### DETAILED DESCRIPTION

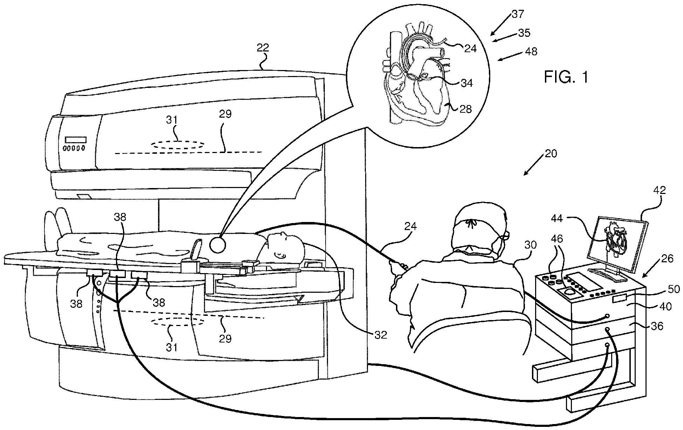

Reference is now made to Fig. 1, which is a schematic, pictorial illustration of a system 20 for enhanced magnetic resonance imaging (MRI), according to an embodiment of the present invention. System 20 comprises an MRI scanner 22, a probe 24, such as a catheter, and a control console 26. As described hereinbelow, probe 24 is configured to provide enhanced MRI images of tissue typically comprised in a body cavity of a patient 32, although this is typically not the only function of the probe. For example, probe 24 may also be used for mapping electrical potentials in a certain chamber of a heart 28 of patient 32, using an electrode 35 in a distal end 34 of the probe. In some embodiments, probe 24 may be used for additional purposes, such as for performing cardiac ablation. Further alternatively, probe 24 may be used, *mutatis mutandis,* for other therapeutic and/or diagnostic functions in the heart or in other body organs.

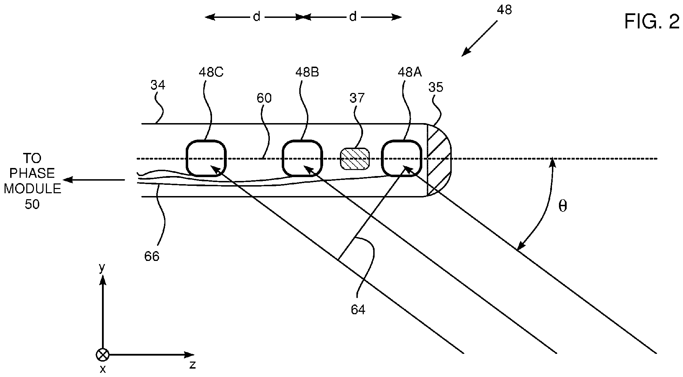

An operator 30, such as a cardiologist, inserts probe 24 through the vascular system of patient 32 so that distal end 34 of the probe enters a body cavity, herein assumed to be the cardiac chamber to be imaged. Distal end 34 is illustrated and explained in more detail with respect to Fig. 2. Console 26 uses magnetic position sensing to determine orientation and location coordinates of distal end 34 inside heart 28. For the sensing, console 26 operates a driver circuit 36 that drives field generators 38, which typically comprise coils placed at known positions, e.g., below the patient's torso. A magnetic field transducer 37 that acts, and is also herein referred to, as a position sensor is installed in distal end 34. Position sensor 37 generates electrical signals in response to the magnetic fields from the coils, thereby enabling console 26 to determine the position, i.e., the orientation and location of distal end 34, within the chamber, with respect to generators 38 and patient 32.

Although in the present example system 20 measures the position, i.e., the orientation and location, of distal end 34 using magnetic-based sensors, other position tracking techniques may be used (e.g., impedance-based techniques) for measuring the position coordinates. Magnetic position tracking techniques are described, for example, in U.S. Patents 5,391,199, 5,443,489, 6,788,967, 6,690,963, 5,558,091, 6,172,499 6,177,792. Impedance-based position tracking techniques are described, for example, in U.S. Patents 5,983,126, 6,456,864 and 5,944,022.

MRI scanner 22 comprises magnetic field coils 29, including field gradient coils, which together generate a spatially variant magnetic field B(x,y,z). The spatially variant magnetic field provides spatial localization for radio frequency (RF) signals generated in the scanner. In addition, the scanner comprises transmit/receive coils 31. In a transmit mode coils 31 radiate RF energy to patient 32, the RF energy interacting with the nuclear spins of the patient's tissue and thereby realigning the magnetic moments of the nuclei away from their equilibrium positions. In a receive mode, coils 31 detect RF signals received from the patient's tissue as the tissue nuclei relax to their equilibrium state. The frequency of the signals generated by the relaxation of nuclei in a given region, the Larmor frequency, is directly proportional to the magnetic field at the region, with a constant of proportionality given by the gyromagnetic ratio $\gamma$ of the nuclei. Thus, for hydrogen nuclei, equation (1) applies:

$$f(x, y, z) = \frac{\gamma}{2\pi} \cdot B(x, y, z) \qquad (1)$$

where f(x,y,z) is the frequency radiated by the relaxing hydrogen nuclei from a point (x,y,z),
B(x,y,z) is the magnetic field at the point, and

$\dfrac{\gamma}{2\pi}$ is equal to approximately 42.6 MHz·T$^{-1}$.

A processor 40 operates scanner 22 by using circuitry to control coils 29, including forming required magnetic field gradients, as well as other circuitry to operate transmit/receive coils 31. Processor 40 acquires MRI data of the patient's heart 28, or at least of the cardiac chamber to be imaged, using signals received by coils 31. In addition, the processor acquires extra MRI data from signals generated in receive coils 48 in distal end 34, using a phase module 50. Receive coils 48, and the acquisition of the extra MRI data using module 50, are described below. The combined MRI data is typically collected at multiple phases of the cardiac cycle of heart 28, often (although not necessarily) over at least one cardiac cycle. Using the data, processor 40 displays an image 44 of heart 28 to operator 30 on a display 42. In some embodiments, operator 30 can manipulate image 44 using one or more input devices 46.

Processor 40 typically comprises a general-purpose computer, which is programmed in software to carry out the functions that are described herein. The software may be downloaded to processor 40 in electronic form, over a network, for example, or it may be provided on non-transitory tangible media, such as optical, magnetic or electronic memory media. Alternatively, some or all of the functions of processor 40 may be carried out by dedicated or programmable digital hardware components, or by using a combination of hardware and software elements.

In addition to processor 40 using received signals comprising values of f(x,y,z), other factors, such as the rates of decay of the nuclei relaxing to their equilibrium state, as well as parameters of the transmitted RF fields exciting the nuclei into their non-equilibrium state, are used by the processor in generating an image of the patient. Such factors will be apparent to those having skill in the magnetic resonance imaging art.

A typical MRI system has a main magnet which generates a magnetic field between approximately 0.5T and approximately 3T, although fields outside these values are possible. As described above, a spatial gradient is applied to the main magnetic field so as to provide spatial localization of the generated RF signals. For clarity, in the description herein the main magnetic field is assumed to be 2T, and those having ordinary skill in the art will be able to adapt the description, *mutatis mutandis,* for main fields other than 2T. The Larmor frequency of hydrogen nuclei in a field of 2T is approximately 85 MHz, and in the description this value is assumed to be the precession frequency of hydrogen nuclei as they relax to their equilibrium state, and of the radio frequency energy radiated by the relaxing nuclei. In system 20 the radiated energy is detected by coils 31 and coils 48.

In free apace an electromagnetic wave of frequency 85 MHz has a wavelength of approximately 3.5 m. However, the wavelength in a patient environment, assuming the patient is formed mainly of water, is reduced because of the relative permittivity of the water. For a relative permittivity equal to 70 (the approximate value for water at a normal patient temperature and at the frequencies considered here), the wavelength of an 85 MHz electromagnetic wave is approximately 42 cm.

System 20 can be realized as the CARTO XP EP Navigation and Ablation System, available from Biosense Webster, Inc., 3333 Diamond Canyon Road, Diamond Bar, CA 91765, suitably modified to execute the procedures described herein.

Fig. 2 is a schematic figure illustrating distal end 34 in cross-section, according to an embodiment of the present invention. For clarity and simplicity in the following description, distal end 34 has been drawn with respect to a set of xyz orthogonal axes, where the plane of the paper corresponds to a yz plane. The distal end is assumed to be generally cylindrical, and by way of example is assumed to have an axis of symmetry 60 parallel to a z axis. Those with ordinary skill in the art will be able to adapt the description herein for distal ends that may not be circular in cross-section, and/or may have a curved shape, such as in "lasso" catheters produced by Biosense Webster, Inc.

Distal end 34 comprises electrode 35 and transducer 37, and also comprises an array of generally similar planar receive coils 48, which as required are differentiated herein by having a letter appended as a suffix to identifying numeral 48. Array of coils 48 may comprise any convenient integral number of coils greater than one, and three such coils are illustrated in Fig. 2. In the embodiment described herein, coils 48 are assumed to be equidistantly spaced, being separated by a distance d from each other. In addition, each coil is assumed to be aligned so that the planes of each coil define a common plane, parallel to the yz plane, and that centers of the coils lie on axis 60 of distal end 34. Each coil is connected by respective cabling 66 to phase module 50, the module being operated by processor 40.

Distal end 34 typically has a diameter of the order of two or three millimeters, so that coils 48 typically have dimensions smaller than this, i.e., coils 48 have dimensions of the order of 1-2 mm. Processor 40 operates the coils, via cabling 66 and phase module 50, as receiving antennas for the radiating electromagnetic energy from hydrogen nuclei relaxing during an MRI procedure. As explained above, the wavelength of the radiating energy is approximately 42 cm, so that because of their dimensions coils 48 act as small loop antennas, responding to the magnetic field of the electromagnetic radiation. Thus, coils 48 have a maximum gain in the plane of the coils, i.e., in the common yz plane of the coils, since radiation emitted by nuclei of tissue located in this plane may have a magnetic field component orthogonal to the plane. Coils 48 have a minimum (theoretically zero) gain orthogonal to the coils, i.e., in an x direction, since nuclei located in tissue on the x-axis emit radiation with a magnetic field component parallel to the plane of the coils.

In embodiments of the present invention, processor 40 uses phase module 50 to operate coils 48 as a phased array of receiving antennas, so that the array has a synthetic aperture. In a first embodiment illustrated in Fig. 2, incoming radiation to the coils, having a wavelength $\lambda$ and making an angle $\theta$ with axis 60, is assumed to be generated sufficiently far from distal end 34 as to be substantially parallel. In this case, a plane wavefront 64 striking coil 48A is ahead by $2\pi \dfrac{d \cdot \cos\theta}{\lambda}$ in phase from the wavefront when it strikes coil 48B. Similarly, the wavefront striking coil 48B is ahead by $2\pi \dfrac{d \cdot \cos\theta}{\lambda}$ from the wavefront striking coil 48C. In order to maximize detection of the incoming radiation, phase module 50 applies a phase delay of $2 \left(2\pi \dfrac{d \cdot \cos\theta}{\lambda}\right)$ to the signal received at coil 48A, and a phase delay of $2\pi \dfrac{d \cdot \cos\theta}{\lambda}$ to the signal received at coil 48B, the phase delays being measured relative to the signal at coil 48C. Thus there is a phase delay $\varphi$ between adjacent coils given by equation (2):

$$\varphi = 2\pi \frac{d \cdot \cos\theta}{\lambda} \qquad (2)$$

In general, for an array of N coils 48 spaced a distance d apart, where N is an integer greater than or equal to 2, module 50 applies (N-1) phase delays equal to

$$(N-1)\left(2\pi \frac{d \cdot \cos\theta}{\lambda}\right), \quad (N-2)\left(2\pi \frac{d \cdot \cos\theta}{\lambda}\right), \quad (N-3)\left(2\pi \frac{d \cdot \cos\theta}{\lambda}\right), \quad \ldots \; 0 \quad \text{to each of}$$

the coils in order to detect radiation making an angle $\theta$ with axis 60. The applied phase delays increase the gain of the array of coils 48 in a direction defined by angle $\theta$, compared to the gain of a single coil, by a factor of N. In addition, the applied phase delays cause the array to reject radiation of wavelength $\lambda$ making angles different from angle $\theta$ with axis 60. Effectively, as N increases the "receiving lobe" of the array narrows and increases in length in the direction defined by angle $\theta$.

In order to detect radiation in line with distal end axis 60 (parallel to the z axis), where $\theta = 0$, module 50, using equation (2), applies an equal phase delay of $2\pi \dfrac{d}{\lambda}$ between adjacent coils. Using the exemplary value for $\lambda$ of 42 cm given above, and assuming a value of d, the physical separation of coils 48 in the distal end, to be 1 cm, module 50 applies a phase delay of $\dfrac{\pi}{21} \cong 9°$ between adjacent coils to detect radiation on the distal end axis.

Again considering equation (2), to detect radiation where $\theta = 90°$, i.e., orthogonal to the z axis, module 50 applies an equal phase delay of 0 between adjacent coils. In other words, module 50 configures all coils to receive at the same phase.

Thus, by selecting the value of the phase delay applied between adjacent coils, module 50 is able to orient the receiving direction of the coils for any incoming radiation that may be considered to be substantially parallel.

The description above has assumed that coils 48 are arrayed along a straight line, and are equally spaced. Those having ordinary skill in the art will be able to adapt the description, *mutatis mutandis,* for substantially parallel radiation that is incoming to an array of coils that are not equally spaced, and/or that are arrayed on a curved line segment, and all such embodiments are assumed to be within the scope of the present invention.

For any given receiving direction of coils 48, the relaxing nuclei of the tissue emit at different frequencies because of the spatially variant magnetic field applied by coils 29 (Fig. 1) as shown by equation (1). By using a combination of equations (1) and (2), processor 40 is thus able to use signals of coils 48, configured to have a selected receiving direction, to isolate the signals from a unique location along the receiving direction. The nuclei in tissue at each location (x,y,z) emit a frequency f(x,y,z), which will have a corresponding wavelength $\lambda$(x,y,z). Use of the two equations avoids any aliasing of tissue locations that might occur if only the receiving direction of coils 48 is considered.

The description above explains how processor 40 is able to isolate signals from a unique location measured with respect to distal end 34. As is also described above, processor 40 is able to measure the location and orientation of distal end 34 with respect to patient 32 using transducer 37. Processor 40 combines the two sets of measurements to reference the unique location generating the signals for coils 48 to patient 32.

Processor 40 combines the signals from the array of coils 48, processed as described above to isolate signals from a unique location, with signals from receive coils 49 to give an enhanced image of the unique location. The unique location is in the region distal tip 48, and the enhancement of the image may comprise an increased resolution, a faster imaging time, and/or an improved tissue differentiation in region of the unique location. The improved differentiation may comprise physical and/or chemical differences of the tissue of the region, such as differences in tissue density and/or differences in chemical composition of the tissue. Alternatively or additionally the physical differences may comprise estimates of relative and/or absolute temperatures of the tissue.

In one embodiment, during a medical procedure performed on patient 32, the image enhancements described above facilitate operator 30 implementing the procedure. For example, if the procedure comprises an ablation of heart tissue, the enhancements may comprise improved measurements of the temperature of the ablated tissue. Such measurement of the temperature of the tissue as it is being ablated allows operator 30 to judge the progression of the ablation.

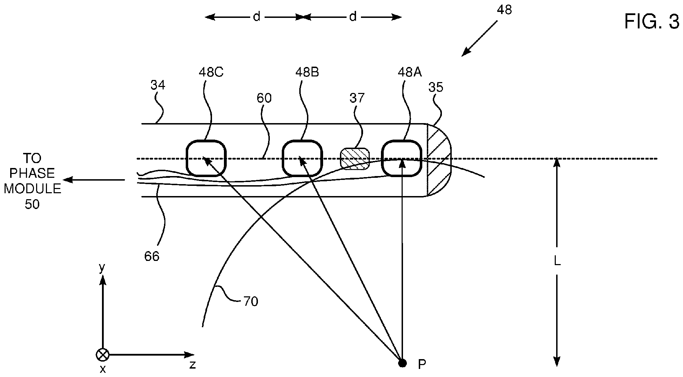

Fig. 3 is a schematic figure illustrating distal end 34 in cross-section, according to an alternative embodiment of the present invention. Apart from the differences described below, the operation of distal end 34 in the alternative embodiment is generally similar to that of the distal end in the configuration described above with reference to Fig. 2, and elements indicated by the same reference numerals in both figures are generally similar in construction and in operation.

In the operation of distal end 34 described with reference to Fig. 2, the array of coils are configured to detect radiation that, as received by the coils and as measured with respect thereto, is substantially parallel. In contrast, in the following description processor 40 configures the operation of coils 48 to detect non-parallel radiation.

For clarity, a position P in the yz plane is assumed to represent nuclei of tissue that emit Larmor frequency radiation as the nuclei relax to their equilibrium state. For simplicity, position P is assumed to be have the same z-value as coil 48A, and to be a distance L (also represented by $d_1$) from the coil.

As illustrated in the figure, a spherical wavefront 70 emitted from position P is ahead of the wavefront when it arrives at second coil 48B by a distance $d_2$ given by equation (3):

$$d_2 = \sqrt{(d^2 + L^2)} - L \qquad\qquad (3)$$

There are thus phase differences $\delta_2$, $\delta_3$ at second and third coils 48B, 48C, compared to the phase at first coil 48A, given by:

$$\left.\begin{aligned} \delta_2 &= 2\pi \frac{\sqrt{(d^2+L^2)}-L}{\lambda(P)} \\ \delta_3 &= 2\pi \frac{\sqrt{((2d)^2+L^2)}-L}{\lambda(P)} \end{aligned}\right\} \qquad\qquad (4)$$

where $\lambda(P)$ is the wavelength of radiation emitted by tissue nuclei at point P.

In general, for a $q^{th}$ coil in an array of coils 48, the phase difference compared to the first coil is given by:

$$\delta_q = 2\pi \frac{\sqrt{((q-1)d)^2+L^2)}-L}{\lambda(P)} \qquad\qquad (5)$$

Using the results of equations (4) and (5), for an array of 1, 2, ... , N coils 48 (where the first coil is assumed to be coil 48A), processor 40 may apply respective phase delays ($\varphi_1$, $\varphi_2$, $\varphi_3$, ... $\varphi_N$ in order to maximize the signal received from position P according to equations (6):

$$\varphi_1 = 2\pi \frac{\sqrt{((N-1)d)^2+L^2}-L}{\lambda(P)}$$

$$\varphi_2 = 2\pi \frac{\sqrt{((N-1)d)^2+L^2}-\sqrt{(d)^2+L^2}}{\lambda(P)}$$

$$\varphi_3 = 2\pi \frac{\sqrt{((N-1)d)^2+L^2}-\sqrt{(2d)^2+L^2}}{\lambda(P)} \qquad\qquad (6)$$

...

$$\varphi_N = 0$$

As is apparent from equations (6), processor 40 applies unequal phase delay differences to coils 48 for situations where the incoming radiation to the coils is non-parallel.

A person having ordinary skill in the art can adapt the analysis described above, *mutatis mutandis,* for any position P in the region of distal end 34, as well as for arrays of coils 48 which are not equally spaced, and/or are not arrayed in a straight line. As described above for incoming parallel radiation (Fig. 2), in the case of incoming non-parallel radiation processor 40 may apply equation (1) to overcome aliasing, so as to uniquely identify a particular region in proximity to the distal end.

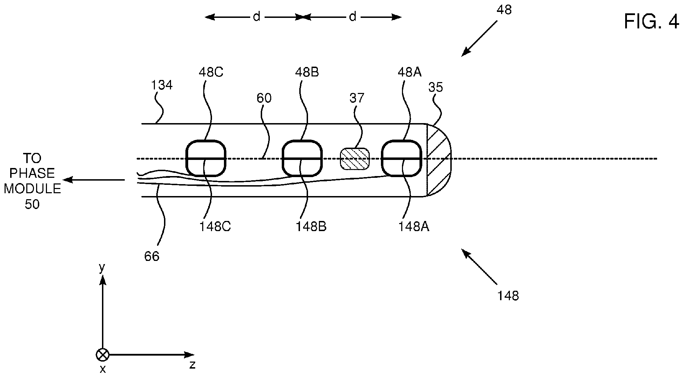

Fig. 4 is a schematic figure illustrating a distal end 134 in cross-section, according to an embodiment of the present invention. Apart from the differences described below, the operation of distal end 134 is generally similar to that of distal end 34 (Figs. 2 and 3), and elements indicated by the same reference numerals in both embodiments are generally similar in construction and in operation.

In distal end 134, a second array of generally similar planar receive coils 148 is located in the distal end. Coils 148 are differentiated, as required, by having a letter appended to the numeral 148. Coils 148 are oriented so that the planes of each coil define a common plane parallel to an xz plane. This is in contrast to coils 48 which are oriented parallel to the yz plane.

In one embodiment, the number of coils 148 in the second array is the same as the number of coils 48, and the centers of coils 148 are arranged to coincide with the centers of coils 48. However, other arrangements of coils 148 are possible, such as having differing numbers of coils in the two arrays, and/or having the coils of the second array spaced or positioned differently from coils 48 of the first array, and all such arrangements are included within the scope of the present invention.

Processor 40 operates coils 148 substantially as described above for coils 48 (Figs. 2 and 3), applying phase delays to the signals received by coils 148 so as to maximize the signals from a selected location in proximity to distal end 134, or from a selected direction with respect to the end. Because coils 148 are oriented to lie in an xz plane, nuclei of tissue in the xz plane containing the coils generate maximum signals at the coils. Thus, applying specific phase delays to the two arrays of coils, coils 48 and coils 148, allows the processor to select and receive signals from the complete three-dimensional region surrounding distal tip 134. As described above, processor 40 may apply equation (1) to overcome any aliasing, so as to uniquely identify a particular region in proximity to distal end 134.

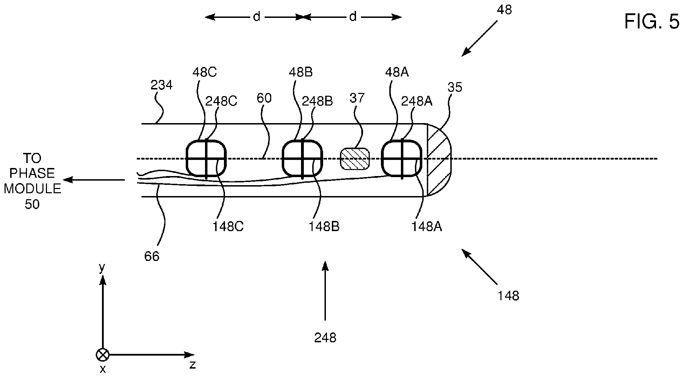

Fig. 5 is a schematic figure illustrating a distal end 234 in cross-section, according to an embodiment of the present invention. Apart from the differences described below, the operation of distal end 234 is generally similar to that of distal ends 134 and 34 (Figs. 2, 3 and 4), and elements indicated by the same reference numerals in the three embodiments are generally similar in construction and in operation.

In distal end 234, a third array of generally similar planar receive coils 248 is located in the distal end. Coils 248 are differentiated, as required, by having a letter appended to the numeral 148. Coils 248 are oriented so that the respective plane of each coil is parallel to an xy plane, and the coils are located so that there is no xy plane common to all coils 248. Typically, the three arrays of coils 48, 148, and 248 are arranged, as illustrated, as sets of three orthogonal coils in the distal end, each set of three coils having a common coil center.

Coils 248 have maximum gains in their respective xy planes (and theoretically zero gain along axis 60). Notwithstanding coils 248 having no common xy plane, it will be apparent to those having ordinary skill in the art that processor 40 can be configured to select and apply differing phase delays to the signals received by coils 248 so as to maximize the signals coming from directions other than axis 60, as well as from locations not on the axis, substantially as described above for coils 48 and 148.

Thus, applying specific phase delays to the three arrays of coils, coils 48, coils 148, and coils 248, allows the processor to select and receive signals from the complete three-dimensional region surrounding distal tip 234. As described above, processor 40 may apply equation (1) to overcome aliasing, so as to uniquely identify a particular region in the vicinity of distal end 234.

The embodiments described above have assumed that magnetic field transducer 37 is installed as a separate component in the distal end of probe 24, and is operated to determine the orientation and location of the distal end. In some embodiments of the present invention, at least some of coils 34, 134, and/or coils 234 are configured to also function as transducer 37, in addition to the functions of the coils described above. For these embodiments, a separate transducer 37 may not be required in the distal end.

The description above has assumed that probe 24 is manually inserted by operator 30 into a body cavity of patient 32. In an alternative embodiment of the present invention, the probe may be inserted robotically into the body cavity of the patient. A robotic drive for a probe is described in U.S. Patent Application 2011/0040150, to Govari et al., and titled Robotic Drive for Catheter. Those having ordinary skill in the art will be able to adapt the description therein, *mutatis mutandis,* to implement a robotic drive for probe 24 so that it is compatible with operation in an MRI environment. Such adaptation includes, for example, replacement of ferromagnetic elements of the drive or modules therein, described in the above-referenced application, with non-magnetic materials such as polyimide based materials. Alternatively or additionally, the adaptation may include replacement of elements using magnetic fields with MRI-compatible equivalent elements. For example, induction or stepper motors may be replaced by air-driven motors having non-magnetic parts.

It will be appreciated that the embodiments described above are cited by way of example, and that the present invention is not limited to what has been particularly shown and described hereinabove.

**Claims**

1. A medical probe (24), comprising:

   a flexible insertion tube having a distal end (34) for insertion into a body cavity;
   an array of spatially separated coils (48) positioned within the distal end; and
   a processor (40), configured to process respective signals generated by the coils (48) in response to magnetic resonance of tissue in the body cavity so as to image the tissue;
   **characterised in that** the processor (40) is further configured to process the signals while applying a phase delay responsive to a separation between the coils (48) so as to maximize signals from a particular direction, or from a particular location.

2. The medical probe according to claim 1, wherein the coils (48) are planar, and wherein respective planes of the coils comprise a common plane.

3. The medical probe according to claim 1, wherein the processor (40) is configured to determine the phase delay responsive to a direction of the tissue with respect to the distal end (34).

4. The medical probe according to claim 1, wherein the processor (40) is configured to determine the phase delay responsive to a location of the tissue with respect to the distal end (34).

5. The medical probe according to claim 1, and comprising a position sensor (37) located in the distal end (34), and wherein the processor (40) is configured to determine a position of the distal end (34) in response to a position signal from the position sensor (37) and to determine the phase delay responsive to the position.

6. The medical probe according to claim 1, wherein at least one of the coils (48) is configured to provide a position signal for the distal end (34), and wherein the processor (40) is configured to determine a position of the distal end (34) in response to the position signal and to determine the phase delay responsive to the position.

7. The medical probe according to claim 1, wherein the spatially separated coils (48) are positioned on a straight line.

8. The medical probe according to claim 1, wherein the spatially separated coils (48) are positioned on a curved line.

9. The medical probe according to claim 1, wherein the coils (48) are separated equidistantly.

10. The medical probe according to claim 1, and comprising a robotic drive compatible with a magnetic resonance imaging (MRI) environment and configured to robotically insert the flexible insertion tube into the body cavity.

11. A medical probe (24) according to claim 1, wherein said coils are first planar coils (48), wherein respective planes of the first planar coils are parallel to a first plane, and wherein the medical probe comprises a second array of spatially separated second planar coils (148) positioned within the distal end (134), wherein respective planes of the second planar coils (148) are parallel to a second plane orthogonal to the first plane; and said processor (40) is configured to process said second signals generated by the second (148) planar coils in response to magnetic resonance of tissue in the body cavity, and to process the second signals while applying a second phase delay responsive to a second separation between the second coils (148), so as to maximize signals from a second particular direction, or from a second particular location, so as to image the tissue.

12. The medical probe according to claim 11, wherein at least one first planar coil (48) and at least one second planar coil (148) have a common center.

13. The medical probe according to claim 11, wherein the respective planes of the first planar coils (48) are common to the first plane.

**Patentansprüche**

1. Medizinische Sonde (24), die Folgendes umfasst:

einen flexiblen Einführschlauch mit einem distalen Ende (34) zum Einführen in einen Körperhohlraum;
ein Array von räumlich getrennten Spulen (48), die in dem distalen Ende positioniert sind; und
einen Prozessor (40), der dazu ausgelegt ist, jeweilige Signale zu verarbeiten, die durch die Spulen (48) als Reaktion auf Magnetresonanz von Gewebe im Körperhohlraum erzeugt werden, um das Gewebe abzubilden; **dadurch gekennzeichnet, dass** der Prozessor (40) ferner dazu ausgelegt ist, die Signale zu verarbeiten, während eine Phasenverzögerung responsiv zu einer Separation zwischen den Spulen (48) angewendet wird, um Signale von einer speziellen Richtung oder von einem speziellen Ort zu maximieren.

2. Medizinische Sonde nach Anspruch 1, wobei die Spulen (48) planar sind, und wobei jeweilige Ebenen der Spulen eine gemeinsame Ebene umfassen.

3. Medizinische Sonde nach Anspruch 1, wobei der Prozessor (40) dazu ausgelegt ist, die Phasenverzögerung responsiv zu einer Richtung des Gewebes bezüglich des distalen Endes (34) zu bestimmen.

4. Medizinische Sonde nach Anspruch 1, wobei der Prozessor (40) dazu ausgelegt ist, die Phasenverzögerung responsiv zu einem Ort des Gewebes bezüglich des distalen Endes (34) zu bestimmen.

5. Medizinische Sonde nach Anspruch 1, umfassend einen Positionssensor (37), der sich im distalen Ende (34) befindet, und wobei der Prozessor (40) dazu ausgelegt ist, eine Position des distalen Endes (34) als Reaktion auf ein Positionssignal vom Positionssensor (37) zu bestimmen und die Phasenverzögerung responsiv zu der Position zu bestimmen.

6. Medizinische Sonde nach Anspruch 1, wobei mindestens eine der Spulen (48) dazu ausgelegt ist, ein Positionssignal für das distale Ende (34) bereitzustellen, und wobei der Prozessor (40) dazu ausgelegt ist, eine Position des distalen Endes (34) als Reaktion auf das Positionssignal zu bestimmen und die Phasenverzögerung responsiv zu der Position zu bestimmen.

7. Medizinische Sonde nach Anspruch 1, wobei die räumlich getrennten Spulen (48) auf einer geraden Linie positioniert sind.

8. Medizinische Sonde nach Anspruch 1, wobei die räumlich getrennten Spulen (48) auf einer gekrümmten Linie positioniert sind.

9. Medizinische Sonde nach Anspruch 1, wobei die Spulen (48) äquidistant getrennt sind.

10. Medizinische Sonde nach Anspruch 1, und umfassend einen robotischen Antrieb, der mit einer Magnetresonanztomographie(MRT)-Umgebung kompatibel ist und dazu ausgelegt ist, den flexiblen Einführschlauch robotisch in den Körperhohlraum einzuführen.

**11.** Medizinische Sonde (24) nach Anspruch 2, wobei die Spulen erste planare Spulen (48) sind, und wobei die medizinische Sonde ein zweites Array von räumlich getrennten zweiten planaren Spulen (148) umfasst, die in dem distalen Ende (134) positioniert sind, wobei jeweilige Ebenen der zweiten planaren Spulen (148) zu einer zweiten Ebene orthogonal zu der ersten Ebene parallel sind; und

der Prozessor (40) dazu ausgelegt ist, die zweiten Signale zu verarbeiten, die durch die zweiten (148) planaren Spulen als Reaktion auf Magnetresonanz von Gewebe im Körperhohlraum erzeugt werden, und die zweiten Signale zu verarbeiten, während eine zweite Phasenverzögerung responsiv zu einer zweiten Separation zwischen den zweiten Spulen (148) angewendet wird, um Signale von einer zweiten speziellen Richtung oder von einem zweiten speziellen Ort zu maximieren, um das Gewebe abzubilden.

**12.** Medizinische Sonde nach Anspruch 11, wobei mindestens eine erste planare Spule (48) und mindestens eine zweite planare Spule (148) eine gemeinsame Mitte aufweisen.

**13.** Medizinische Sonde nach Anspruch 11, wobei die jeweiligen Ebenen der ersten planaren Spulen (48) der ersten Ebene gemein sind.

**Revendications**

**1.** Sonde médicale (24), comprenant :

un tube d'insertion flexible ayant une extrémité distale (34) pour l'insertion dans une cavité corporelle ;
un ensemble de bobines séparées dans l'espace (48) et positionnées à l'intérieur de l'extrémité distale ; et
un processeur (40), configuré pour traiter les signaux respectifs générés par les bobines (48) en réponse à la résonance magnétique du tissu dans la cavité corporelle afin de produire une image du tissu ;
**caractérisé en ce que** le processeur (40) est en outre configuré pour traiter les signaux tout en appliquant un retard de phase en réponse à une séparation entre les bobines (48) de manière à maximiser les signaux provenant d'une direction particulière, ou d'un emplacement particulier.

**2.** Sonde médicale selon la revendication 1, les bobines (48) étant planes, et les plans respectifs des bobines comprenant un plan commun.

**3.** Sonde médicale selon la revendication 1, le processeur (40) étant configuré pour déterminer le retard de phase en réponse à une direction du tissu par rapport à l'extrémité distale (34).

**4.** Sonde médicale selon la revendication 1, le processeur (40) étant configuré pour déterminer le retard de phase en réponse à un emplacement du tissu par rapport à l'extrémité distale (34).

**5.** Sonde médicale selon la revendication 1, et comprenant un capteur de position (37) situé dans l'extrémité distale (34), et le processeur (40) étant configuré pour déterminer une position de l'extrémité distale (34) en réponse à un signal de position provenant du capteur de position (37) et pour déterminer le retard de phase en réponse à la position.

**6.** Sonde médicale selon la revendication 1, au moins une des bobines (48) étant configurée pour fournir un signal de position pour l'extrémité distale (34), et le processeur (40) étant configuré pour déterminer une position de l'extrémité distale (34) en réponse au signal de position et pour déterminer le retard de phase en réponse à la position.

**7.** Sonde médicale selon la revendication 1, les bobines séparées dans l'espace (48) étant positionnées sur une ligne droite.

**8.** Sonde médicale selon la revendication 1, les bobines séparées dans l'espace (48) étant positionnées sur une ligne courbe.

**9.** Sonde médicale selon la revendication 1, les bobines (48) étant séparées de manière équidistante.

**10.** Sonde médicale selon la revendication 1, et comprenant un entraînement robotique compatible avec un environnement d'imagerie par résonance magnétique (IRM) et configuré pour insérer de manière robotisée le tube d'insertion flexible dans la cavité corporelle.

**11.** Sonde médicale (24) selon la revendication 2, lesdites bobines étant des premières bobines planes (48) et, la sonde médicale comprenant un deuxième ensemble de deuxièmes bobines planes (148) séparées dans l'espace et positionnées à l'intérieur de l'extrémité distale (134), les plans respectifs des deuxièmes bobines planes (148) étant parallèles à un deuxième plan orthogonal au premier plan ; et
ledit processeur (40) étant configuré pour traiter lesdits seconds signaux générés par les secondes bobines planes (148) en réponse à la résonance magnétique du tissu dans la cavité corporelle, et pour traiter les seconds signaux tout en appliquant un second retard de phase en réponse à une seconde séparation entre les secondes bobines (148), de manière à maximiser les signaux provenant d'une seconde direction particulière, ou d'un second emplacement particulier, de manière à produire une image du tissu.

**12.** Sonde médicale selon la revendication 11, au moins une première bobine plane (48) et au moins une deuxième bobine plane (148) ayant un centre commun.

**13.** Sonde médicale selon la revendication 11, les plans respectifs des premières bobines planes (48) étant communs au premier plan.

FIG. 1

FIG. 2

FIG. 3

FIG. 4

FIG. 5

TO
PHASE
MODULE
50

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003325475 A
- US 5391199 A
- US 5443489 A
- US 6788967 B
- US 6690963 B
- US 5558091 A
- US 6172499 B
- US 6177792 B
- US 5983126 A
- US 6456864 B
- US 5944022 A
- US 20110040150 A, Govari